# EUROPÄISCHE PATENTSCHRIFT

(11) **EP 0 982 021 B1**
(45) Veröffentlichungstag und Bekanntmachung des Hinweises auf die Patenterteilung: **02.01.2008**
(21) Anmeldenummer: 99115820.5
(22) Anmeldetag: 11.08.1999
(51) Int. Cl.: A61K 8/00, A61K 8/02, A61K 8/30, A61K 8/72, A61Q 5/00, A61Q 5/02, C11D 1/66, C11D 3/37, C11D 17/00

(54) **Verdickungsmittel für tensidhaltige Zusammensetzungen**
Thickening agents for surfactant containing compositions
Agents epaississants pour compositions contenant des agents tensioactifs

(30) Priorität: 14.08.1998 DE 19836808
(43) Veröffentlichungstag der Anmeldung: 01.03.2000
(73) Patentinhaber: BASF Aktiengesellschaft, 67056 Ludwigshafen (DE)
(72) Erfinder: Meffert, Helmut, Dr., 68161 Mannheim (DE); Tiefensee, Kristin, Dr., 67368 Westheim (DE)

(56) Entgegenhaltungen:
- EP-A- 0 128 237
- EP-A- 0 494 022
- WO-A-91/09108
- WO-A-98/31334
- DE-A- 1 770 891
- DE-A- 4 412 790
- US-A- 3 915 921
- US-A- 4 686 254
- US-A- 5 776 882

## Beschreibung

Die vorliegende Erfindung betrifft die Verwendung von hydrophob modifizierten Polymeren, monoethylenisch ungesättigter Carbonsäuren als Verdickungsmittel in Haarwaschmitteln, sowie wäßrige Zusammensetzungen, welche diese Polymere enthalten.

Verdickungsmittel werden in großem Umfang zur Erhöhung der Viskosität von wäßrigen Zubereitungen eingesetzt, beispielsweise auf dem Gebiet der Pharmazie und der Kosmetik. Beispiele für häufig verwendete Verdickungsmittel sind Fettsäurepolyethylenglykolmonoester, Fettsäurepolyethylenglykoldiester, Fettsäurealkanolamide, oxethylierte Fettalkohole, ethoxylierte Glycerinfettsäureester, Celluloseether, Natriumalginat, Polyacrylsäuren sowie Neutralsalze.

Die Verwendung der bekannten Verdickungsmittel ist jedoch, je nach der zu verdickenden Zubereitung, mit Nachteilen verbunden. So kann die Verdickungswirkung und die Salzstabilität der Verdickungsmittel nicht zufriedenstellend und ihre Einarbeitung in die zu verdickende Zubereitung erschwert sein.

US-Patent 3,915,921 (The B.F. Goodrich Company) beschreibt Copolymerisate aus 95-50 Gew.-% monoethylenisch ungesättigten Carbonsäuren und 5-50 Gew.-% eines Acrylsäure- bzw. Methacrylsäuresters eines C₁₀-C₃₀ Fettalkohols. Optional können die Polymere vernetzt sein. Die Copolymerisate werden als Verdicker für Zahnputzmittel und Druckpasten verwendet.

EP 0 268 164 (The B.F. Goodrich Company) beschreibt die Verwendung von vernetzten Copolymerisaten von monoolefinisch ungesättigten Säuren (50-99 Gew.-%) und Alkylester monoolefinisch ungesättigter Säuren (50-1 Gew.-%) (mit Pentaerythrittriallylether vernetzt), die unter dem CTFA-Namen "Acrylates/C₁₀₋₃₀-Alkyl Acrylate Crosspolymer" bekannt sind. Diese werden zur Stabilisierung von O/W-Emulsionen in kosmetischen und pharmazeutischen Zubereitungen, wie z.B. Hautcremes, -lotionen und Gelen verwendet.

In WO 97/21744 (BASF Aktiengesellschaft) werden zwingend vernetzte Copolymere verwendet.

Diese Polymere sind Fällungspolymerisate und stellen freifließende Pulver dar, die nach dem Einrühren in Wasser neutralisiert werden. Dieser Neutralisationsschritt ist notwendig, um die sauren Polymere in die Carboxylate zu überführen, die letztlich für die Viskosität verantwortlich sind.

Es ist bekannt, daß diese vernetzten (hydrophob modifizierten) Polyacrylsäuren in neutralisiertem Zustand sehr empfindlich auf Salz reagieren. Die Viskosität bricht zusammen. Deshalb ist es unüblich, diese Polymere in Shampoo-Formulierungen als Viskostätsgeber einzusetzen. Aufgrund der dort vorliegenden Salzkonzentrationen (Tenside, Tensid-Gemische, NaCl als Verunreinigung in Tensiden) kann sich keine Viskosität ausbilden. Bei Anwesenheit von kationischen Hilfsstoffen kommt es zur Komplexbildung und Niederschlag.

EP 0128 237 (The B.F. Goodrich Company) beschreibt schwach vernetzte Copolymere (0,1 bis 1,0 Gew.-%) aus monoethylenisch ungesättigten Carbonsäuren (95,5 bis 98,9 Gew.-%) und Estern dieser Carbonsäuren (1 bis 2,5 Gew.-%) für die Verwendung als Verdicker in einer Druckpaste.

US-Patent 4,432,881 (Dow Chemical Company) beschreibt Copolymere aus wasserlöslichen Monomeren wie z.B. Acrylamid, Acrylsäure u.a., vorzugsweise die Kombinationen davon, und N-Alkylacrylamide und Acrylsäureester. Die Verhältnisse hydrophil/hydrophob-Anteil sind 98:2 mol-% bis 99,995:0,005 mol-%, vorzugsweise 99:1 mol-% bis 99,9:0,1 mol-%. Molekulargewichte werden zwischen 200000-5000000 g/mol, vorzugsweise zwischen 800000-2500000 g/mol angegeben. Verwendet werden die erhaltenen Polymere als dispergierbare hydrophobe Verdicker, eingesetzt in Formulierungen enthaltend die beschriebenen Polymere, eine nicht ionische oberflächenaktive Substanz (HLB 2-15) und ein anorganische Salz zur Viskositätssteigerung von Wasser.

In US-Patent 4,395,524 (Rohm und Haas Company) wird die Copolymerisation von hydrophilen Komponenten (z.B. Acrylamid, Acrylsäure, N-Vinylpyrrolidon u.a.) mit N-Alkylacrylamiden beschrieben (Alkyl = C₁₀ bis C₃₆, vorzugsweise C₁₂ bis C₂₂). Die Copolymerisation wird als Fällungspolymerisation oder Polymerisation in Lösung durchgeführt. Das Molekulargewicht der beschriebenen Polymere ist M_{W} >30000 g/mol. Die so erhaltenen Polymere werden als Verdicker von wäßrigen Systemen, Sedimentationsstabilisatoren, oberflächenaktive Stoffe oder Dispergiermittel verwendet.

WO 98/31334 beschreibt Haarkonditioniermittel, die keine Salze enthalten, nicht klar sind und wasserunlösliche Komponenten wie Silikonöl und Fettalkohole als aktive Komponente enthalten.

Die Aufgabe der Erfindung ist einen Verdicker zur Verfügung zu stellen, der sich problemlos in eine Formulierung einrühren läßt. Die Formulierung enthält mindestens ein Alkyl- oder Alkenylpolyglykosids. Solche Formulierungen werden unter anderem in der kosmetischen Industrie verwendet, besonders bei Haarwaschmitteln, insbesondere bei Shampoos. Die resultierenden Formulierung sollte dabei klar und stabil sein und durch den Zusatz der erfindungsgemäßen Polymere verdicken.

Überraschenderweise wurde nun gefunden, daß neutralisierte hydrophob modifizierte Polymerisate aus monoethylenisch ungesättigten Carbonsäuren, wie in den Ansprüchen definiert, als Verdickungsmittel für tensid-haltige Zusammensetzungen z.B. in milden Shampooformulierungen, insbesondere für Zusammensetzungen, welche ein Alkyl- oder Alkenylpolyglycosid enthalten, hervorragend geeignet sind. Die erfindungsgemäßen Formulierungen sind klar. Die oben genannten Nachteile können dabei nicht beobachtet werden.

Die Aufgabe wird erfindungsgemäß mit Copolymeren gelöst, die mindestens 50 Gew.-% und höchstens 99 Gew.-% monoethylenisch ungesättigte Carbonsäure enthalten. Bevorzugt sind Polymerisate mit mindestens 58 Gew.-% und maximal 99 mol-% monoethylenisch ungesättigte Carbonsäure, ganz besonders bevorzugt sind Polymere mit einem Gehalt an monoethylenisch ungesättigten Carbonsäure von 65 Gew.-% bis 99 Gew.-%.

Die Polymerisate werden dadurch hergestellt, daß man die entsprechenden Monomeren, die jeweils mindestens 50 Gew.-% monoethylenisch ungesättigte Carbonsäure enthalten, radikalisch polymerisiert.

Als hydrophobe Komponente werden mindestens 1, höchstens 50 Gew.-% eines a) monoethylenisch ungesättigten Carbonsäureesters mit einem gesättigten C₈- bis C₃₀-Alkohol, bevorzugt einem C₈- bis C₁₈-Alkohol, eingesetzt (z.B. Octylacrylat, 2-Ethylhexylacrylat, Nonylacrylat, Decylacrylat, Laurylacrylat, Myristylacrylat, Cetylacrylat, Stearylacrylat, Behenylacrylat, Hexylmethacrylat, Octylmethacrylat, Nonylmethacrylat, Decylmethacrylat, Laurylmethacrylat, Myristylmethacrylat, Cetylmethacrylat, Stearylmethacrylat, Behenylmethacrylat, tert.-Butylcyclohexylacrylat). Besondere Bedeutung kommt hierbei den Acryl- bzw. Methacrylsäureestern der Fettalkohole mit 8 bis 18 C-Atomen zu.

Als Alternative zu den monoethylenisch ungesättigten Carbonsäureestern können b) auch N-Alkyl- oder N,N-Dialkylsubstituierte Carbonsäureamide verwendet werden, wobei es sich bei den Alkylresten unabhängig voneinander um aliphatische oder cycloali-phatische Alkylreste mit mindestens 8 bis 18 Kohlenstoffatomen handelt (z.B. N-Stearylacrylamid, N-Stearylmethacrylamid, N-(1-Methyl)-undecylacrylat, N-(1-Methyl)-undecylmethacrylat, N-Dodecylacrylamid, N-Dodecylmethacrylamid, N-Octylacrylamid, N-Octylmethacrylamid, N,N-Dioctylacrylamid, N,N-Dioctylmethacrylamid, N-Cetylacrylamid, N-Cetylmethacrylamid, N-Dodecylacrylamid, N-Dodecylmethacrylamid, N-Myristylacrylamid, N-Myristylmethacrylamid, N-(2-Ethyl)-hexylacrylamid, N-(2-Ethyl)-hexylmethacrylamid.

Als weitere Alternative zu den monoethylenisch ungesättigten Carbonsäureestern können c) auch Vinylester langkettiger aliphatischer Carbonsäuren (C₈ bis C₃₀-Carbonsäuren) eingesetzt werden. Bevorzugt finden Vinylester von C₈ bis C₁₈-Carbonsäuren Verwendung.

Ferner können eingesetzt werden d) Alkyl-vinylether, bevorzugt mit C₈ bis C₁₈-Alkylresten.

Selbstverständlich können auch Mischungen aus zwei oder mehreren Carbonsäureestern, Carbonsäureamiden, Vinylestern oder Alkylvinylethern eingesetzt werden, solange die Summe der Anteile dieser Comonomere nicht 50 Gew.-% überschreitet.

Als hydrophile Komponente können folgende copolymerisierbaren monoethylenisch ungesättigte Carbonsäuren (oder auch Mischungen derselben) verwendet werden (50 bis 99 Gew.-%, bevorzugt 58 bis 99 Gew.-%, besonders bevorzugt 65 bis 99 Gew.-%):

Monoethylenisch ungesättigte Carbonsäuren mit 3 bis 8 C-Atomen wie Acrylsäure, Methacrylsäure, Dimethacrylsäure, Ethacrylsäure, Maleinsäure, Citraconsäure, Methylenmalonsäure, Allylessigsäure, Vinylessigsäure Crotonsäure, Fumarsäure, Mesaconsäure und Itaconsäure, sind bevorzugt.

Aus dieser Gruppe von Monomeren verwendet man vorzugsweise Acrylsäure, Methacrylsäure, Maleinsäure oder Mischungen der genannten Carbonsäuren.

Die monoethylenisch ungesättigten Carbonsäuren können in Form der freien Säure und - soweit vorhanden - der Anhydride oder in partiell oder in vollständig neutralisierter Form bei der Copolymerisation eingesetzt werden. Um diese Monomeren zu neutralisieren, verwendet man vorzugsweise Alkalimetall- oder Erdalkalimetallbasen, Ammoniak oder Amine, z.B. Natronlauge, Kalilauge, Soda, Pottasche, Natriumhydrogencarbonat, Magnesiumoxid, Calciumhydroxid, Calciumoxid, gasförmiges oder wäßriges Ammoniak, Triethylamin, Ethanolamin, Diethanolamin, Triethanolamin, Morpholin, Diethylentriamin oder Tetra-ethylenpentamin.

Weitere geeignete Comonomere sind beispielsweise die C₁-C₄-Ester, N-C₁-C₄-Alkyl- oder N,N-C₁-C₄-Dialkkyl substituierte Amide, wobei die Alkylreste des Dialkylamids gleich oder verschieden sein können, und Nitrile der oben angegebenen Carbonsäuren, z.B. Acrylsäuremethylester, Acrylsäureethylester, Methacrylsäuremethylester, Methacrylsäureethylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäurediethylester, 2-Ethylhexylacrylat, 2-Ethylhexylmethacrylat, Acrylamid, Methacrylamid, N-Dimethylacrylamid, N-tert.-butylacrylamid, Acrylnitril, Methacrylnitril, Dimethylaminoethylacrylat, Diethylaminoethylacrylat, Diethylaminoethylmethacrylat sowie die Salze der zuletzt genannten Monomeren mit Carbonsäuren oder Mineralsäuren sowie die quaternierten Produkte. Die jeweiligen Alkylgruppen der Ester und Amide können Hydroxy substituiert sein.

Außerdem eignen sich als andere copolymerisierbare Monomere Acrylamidoglycolsäure, Vinylsulfonsäure, Allylsulfonsäure, Methallylsulfonsäure, Styrolsulfonsäure, Acrylsäure-(3-sulfopropyl)ester, Methacrylsäure(3-sulfopropyl)ester und Acrylamidomethylpropansulfonsäure sowie Phosphonsäuregruppen enthaltende Monomere, wie Vinylphosphonsäure, Allylphosphonsäure und Acrylamidomethanpropanphosphonsäure.

Weitere geeignete copolymerisierbare Verbindungen sind N-Vinylpyrrolidon, N-Vinylcaprolactam, N-Vinylimidazol, N-Vinyl-2-methylimidazol, N-Vinyl-4-methylimidazol, Diallylammoniumchlorid, Vinylacetat und Vinylpropionat. Es ist selbstverständlich auch möglich, Mischungen der genannten Monomeren einzusetzen.

Die monoethylenisch ungesättigte Carbonsäure und die hydrophobe Komponente oder Komponenten und, falls vorhanden, die weiteren geeigneten Monomeren ergänzen sich zu 100 %.

Die Herstellung der Copolymerisate erfolgt nach bekannten Verfahren, z.B. der Lösungs-, Fällungs-, oder umgekehrte Suspensionspolymerisation unter Verwendung von Verbindungen, die unter den Polymerisationsbedingungen Radikale bilden.

Die Polymerisationstemperaturen liegen üblicherweise in dem Bereich von 30°C bis 200°C, vorzugsweise 40°C bis 140°C. Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, z.B. Natriumsulfit, Natriumbisulfit, Natriumformaldehydsulfoxilat und Hydrazin.

Die Copolymeren besitzen K-Werte von mindestens 10 und sind im wesentlichen nicht quervernetzt. Vorzugsweise besitzen sie einen K-Wert von 12 bis 70, besonders bevorzugt 15 bis 50. Die K-Werte werden bestimmt nach H. Fikentscher, Cellulose-Chemie, Band 13, 58 bis 64 und 71 bis 74 (1932) in 0,1 molarer wäßriger NaCl-Lösung bei 25°C, bei Konzentrationen, die je nach K-Wert-Bereich zwischen 0,1 % und 5 % liegen.

Gegenstand der vorliegenden Erfindung sind auch wäßrige Zusammensetzungen, die wenigstens eine neutralisierte bzw. teilweise neutralisierte hydrophob modifizierte polymere Carbonsäure oben beschriebener Natur und wenigstens ein Tensid enthalten.

Gegenstand der Erfindung sind weiterhin wäßrige Zusammensetzungen, die enthalten:
a) wenigstens ein Alkyl- oder Alkenylpolyglykosid, insbesondere ein C₈-C₁₈-Alkyl- oder C₈-C₁₈-Alkenylpolyglykosid,
b) wenigstens eine hydrophob modifizierte polymere Carbonsäure oben beschriebener Zusammensetzung,
c) gegebenenfalls ein weiteres, von a) verschiedenes Tensid und
d) gegebenenfalls wenigstens ein Neutralsalz und
e) weitere Hilfsstoffe wie Konservierungsmittel, Emulgatoren, Parfümöle, Pflegestoffe wie Panthenol, Collagen, Vitamine, Eiweißhydrolysate, Stabilisatoren, pH-Wert-Regulatoren, Farbstoffe, Perlglanzmittel, Konsistenzgeber, Silikone, Feuchthaltemittel, Rückfetter, Salze, Säuren (beispielsweise Milchsäure, Zitronensäure) und weitere übliche Additive.

Bei den Polyglykosiden handelt es sich vorzugsweise um Polyglucoside, die ein durch Acetalisierung von Glucose mit Fettalkoholen erhaltenes Homologengemisch darstellen. Die mittlere Anzahl an Glucoseeinheiten pro Molekül liegt im Bereich von 1 bis 3.

Ein bevorzugtes Alkypolyglucosid (APG) ist Plantaren^{®}, bei dem 1 bis 7 Glucoseeinheiten glycosidisch mit einem Fettalkohol (zumeist 12 C-Atome) verknüpft sind. Vorzugsweise sind 2 Glucose Einheiten verknüpft.

Der Anteil an Polyglucosiden liegt vorzugsweise bei maximal 60 Gew.-%.

Die oben beschriebenen Polymere sind in den Zusammensetzungen im Allgemeinen in einer Menge von mindestens 0,2 Gew.-%, bevorzugterweise bis 30 Gew.-%, besonders bevorzugt in einer Menge von mindestens 0,5 bis 20 Gew.-%, bezogen auf das Gesamtgewicht des Mittels, enthalten.

Als Tensid können in den erfindungsgemäßen Mitteln anionische, nichtionische, kationische und amphotere Tenside enthalten sein.

Geeignete anionische Tenside sind beispielsweise Alkylsulfate, Alkylethersulfate, Alkylarylsulfonate, Alkylsuccinate, Alkylsulfosuccinate, N-Alkoylsarkosinate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxideinheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate, oder -dipropionate.

Beispielsweise können Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol.

Ferner sind Alkylaminoxide, Mono- oder Dialkylalkanolamide, Fettsäurester von Polyethylenglykolen, ethoxylierte Fettsäureamide, Alkylpolyglykoside oder Sorbitanetherester geeignet.

Außerdem können die Formulierungen (z.B. Shampooformulierungen) übliche kationische Tenside enthalten, wie z.B. quaternäre Ammoniumverbindungen, beispielsweise Cetyltrimethylammoniumchlorid.

Zusätzlich können auch weitere übliche kationische Polymere eingesetzt werden, so z.B. Copolymere aus Acrylamid und Dimethyldiallylammoniumchlorid (Polyquaternium-7), kationische Cellulosederivate (Polyquaternium-10), Guar-hydroxypropyltrimethylammoniumchlorid (INCI: Hydroxypropyl Guar Hydroxypropyltrmonium Chloride), Copolymere aus N-Vinylpyrrolidon und quaternisiertem N-Vinylimidazol (Polyquaternium-16, -44, -46) und andere.

Zur zusätzlichen Verdickung können die erfindungsgemäßen Zusammensetzungen ein Neutralsalz, insbesondere Natriumsulfat und vorzugsweise Natriumchlorid, enthalten. Das Neutralsalz ist im Allgemeinen in einer Menge von 0,1 bis 10 Gew.-%, insbesondere 0,5 bis 5 Gew.-%, enthalten.

Als weitere zusätzliche Verdicker in den Formulierungen können z.B. PEG-55, Propylene Glycol Oleate, PEG-120 Methyl Glucose Dioleate und andere verwendet werden.

Darüber hinaus können die erfindungsgemäßen Zusammensetzungen übliche, dem Fachmann bekannte Hilfs- und Zusatzstoffe enthalten, beispielsweise Konservierungsmittel, Emulgatoren, Parfümöle, Pflegestoffe wie Panthenol, Collagen, Vitamine, Eiweißhydrolysate, Stabilisatoren, pH-Wert-Regulatoren, Farbstoffe, Perlglanzmittel, Konsistenzgeber, Silikone, Feuchthaltemittel, Rückfetter, Salze, Säuren (beispielsweise Milchsäure, Zitronensäure) und weitere übliche Additive.

Die erfindungsgemäß verwendeten Polymere können auch mit herkömmlichen in der Kosmetik üblichen Polymeren abgemischt werden, falls ganz spezielle Eigenschaften eingestellt werden sollen.

Weitere geeignete Kosmetikpolymere (z.B. in Shampooformulierungen) sind z.B. kationische Polymere mit der Bezeichnung Polyquaternium nach INCI, z.B. Copolymere aus Vinylpyrrolidon/N-Vinylimidazoliumsalzen (Luviquat^{®} FC, Luviquat^{®} HM, Luviquat^{®} MS, Luviquat^{®} Care), Copolymere aus N-Vinylpyrrolidon/Dimethylaminoethylmethacrylat, quaternisiert mit Diethylsulfat (Luviquat^{®} PQ11), Copolymere aus N-Vinylcaprolactam/N-Vinylpyrrolidon/N-Vinylimidazoliumsalzen (Luviquat^{®} Hold); kationische Cellulosederivate (Polyquaternium-4 und -10), Acrylamidcopolymere (Polyquaternium-7), kationische Guar Gum Derivate.

Als weitere geeignete Kosmetikpolymere (z.B. in der Haarkosmetik)sind auch neutrale Polymere geeignet wie Polyvinylpyrrolidone, Copolymere aus N-Vinylpyrrolidon und Vinylacetat und/oder Vinylpropionat, Polysiloxane, Polyvinylcaprolactam und Copolymere mit N-Vinylpyrrolidon, Polyethylenimine und deren Salze, Polyvinylamine und deren Salze, Cellulosederivate, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane oder Silikonharze.

Jeweils bezogen auf das Gesamtgewicht enthalten die erfindungsgemäßen wäßrigen Zusammensetzungen im Allgemeinen:
- 0,2 bis 20 Gew.-%, vorzugsweise 0,5 bis 15 Gew.-% wenigstens ein Polymer oben beschriebener Zusammensetzung,
- 0 bis 50 Gew.-%, wenigstens eines Tensids und
- 0,5 bis 50 Gew.-%, wenigstens eines Alkyl- oder Alkylenpolyglykosids,
bezogen auf das Gesamtgewicht der wäßrigen Formulierung.

Gemäß einer bevorzugten Ausführungsform enthalten die erfindungsgemäßen wäßrigen Zusammensetzungen:
a) 0,2 bis 20 Gew.-%, vorzugsweise 0,5 bis 15 Gew.-% wenigstens eines Polymers oben beschriebener Zusammensetzung;
b) 1 bis 50 Gew.-%, wenigstens eines anionischen Tensids, insbesondere eines Alkyethersulfates;
c) 0,5 bis 50 Gew.-%, wenigstens eines C₈-C₁₈-Alkyl- oder C₈-C₁₈-Alkenylpolyglykosids;
d) 0 bis 5 Gew.-%, vorzugsweise 0,5 bis 3 Gew.-% wenigstens eines Neutralsalzes,
bezogen auf das Gesamtgewicht der wäßrigen Formulierung.

Die Herstellung dieser erfindungsgemäßen Zusammensetzungen erfolgt in üblicher Weise, wobei die beschriebenen Polymere als solche oder als wäßrige Lösung eingesetzt werden können. Im Allgemeinen wird das Verdickungsmittel in die wäßrige Tensid-Mischung eingerührt. Hilfsstoffe können problemlos in die verdickte Mischung eingerührt werden.

Bei den erfindungsgemäßen Zusammensetzungen handelt es sich insbesondere um kosmetische oder pharmazeutische Zusammensetzungen. Bevorzugte kosmetische Zusammensetzungen sind Haarpflegemittel, insbesondere Shampoos.

Die nachfolgenden Beispiele sind Referenzbeispiele, welche nicht unter die Ansprüche fallen.

### Beispiele

Folgende Verdicker wurden verwendet:

| Polymer | K-Wert *) | Acrylsäure/Stearylmethacrylat (Gew.-%) |
|---|---|---|
| 1 | 28 | 70/30 |
| 2 | 15 | 70/30 |
| 3 | 25 | 78/22 |
| 4 | 30 | 65/35 |

| | | |
|---|---|---|
| *) Die K-Werte wurden bestimmt nach H. Fikentscher, Cellulose-Chemie, Band 13, 58 bis 64 und 71 bis 74 (1932) in 0,1 molarer wäßriger NaCl-Lösung bei 25°C mit einer 1 %igen Polymerlösung | | |

Nachstehende Tensid-Mischungen wurden mit unterschiedlichen Polymerisaten als Verdicker formuliert; die detaillierten Zusammensetzungen sind in Tabelle 1 zusammengefaßt:

### Rezeptur 1

| | |
|---|---|
| Decylglucosid | 8 % |
| Sodium laureth sulfate | 4 % |
| Verdicker | q.s. |
| NaCl | q.s. |
| Citronensäure | q.s. |
| Wasser | ad 100 % |

### Rezeptur 2

| | |
|---|---|
| Sodium/Mg laureth sulfate | |
| Sodium/Mg Oleth sulfate | 10 % |
| Lauryl glucosid | 4 % |
| Cocoamidopropylbetain | 3 % |
| Polyquaternium-16 | 2 % |
| Verdicker | q.s. |
| Citronensäure | q.s. |
| Wasser | ad 100 % |

### Rezeptur 3

| | |
|---|---|
| Laurylglucosid | 13 % |
| Cocoamidopropylbetain | 5 % |
| Cocotrimonium Methosulfate | 2 % |
| Hydrolyzed Wheat Gluten | 2 % |
| Verdicker | q.s. |
| Citronensäure | q.s. |
| Konservierungsmittel | 0,1 % |
| Wasser | ad 100 % |

**Tabelle 1**

| Rezeptur | Polymer | Polymergehalt [%] | NaCl - Konz. [%] | Viskosität [mPas] ¹⁾ | Aussehen | pH-Wert |
|---|---|---|---|---|---|---|
| 1 | 1 | 3 | 3 | 3 500 | Klar | 6,1 |
| | | 5 | 3 | 12 000 | Klar | 6,1 |
| | | 5 | 1 | 7 900 | Klar | 6,2 |
| | | 5 | 0 | 1 000 | klar | 6,6 |
| | 2 | 3 | 3 | 1 200 | klar | 5,9 |
| | | 5 | 3 | 7 000 | klar | 6,0 |
| | 3 | 3 | 3 | 3 700 | klar | 5,8 |
| | | 5 | 3 | 10 800 ²⁾ | klar | 6,1 |
| 2 | 1 | 3 | 0 | 40 000 ³⁾ | klar | 6,5 |
| | | 1 | 0 | 12 700 | klar | 6,5 |
| 3 | 1 | 0, 5 | - | 10 100 ²⁾ | klar | |

| | | | | | | |
|---|---|---|---|---|---|---|
| ¹⁾ Bookfield, 10 UpM, 23°C, Spindel 3 ²⁾ dto., Spindel 4 ³⁾ dto., Spindel 6 | | | | | | |

**Tabelle 2**

| Rezeptur | Polymer | Polymergehalt [%] | NaCl - Konz. [%] | Viskosität [mPas] ¹) | Aussehen | pH-Wert |
|---|---|---|---|---|---|---|
| 1 | Pemulen TR 1*) | 3 | 3 | 40 600 ¹⁾ | weiß, fest, schleimig | 5,9 |
| | Dto. | 1 | - | 15 000 ²⁾ | Weiß, schleimig | 5,8 |
| 2 | Pemulen TR 1 | 3 | - | 52 000 ³⁾ | Weiß, fest, schleimig | 6,1 |

| | | | | | | |
|---|---|---|---|---|---|---|
| *) Produkte der B.F. Goodrich 1) Bookfield, 10 UpM, 23°C, Spindel 6 2) dto., Spindel 4 3) dto., Spindel 7 | | | | | | |

## Patentansprüche

1. Verwendung von neutralisierten oder teilneutralisierten Copolymerisaten erhältlich aus
A) 50 bis 99 Gew.-% monoethylenisch ungesättigter Carbonsäure und
B) 1 bis 50 Gew.-% mindestens einem Comonomeren ausgewählt aus den Gruppen a) bis d) oder auch Mischungen von verschiedenen Monomeren aus den Gruppen a) bis d)
a) monoethylenisch ungesättigte Carbonsäureester mit einem gesättigten C₈- bis C₃₀-Alkohol
b) N-Alkyl- oder N,N-Dialkylsubstituierte Carbonsäureamide, wobei es sich bei den Alkylresten unabhängig voneinander um aliphatische oder cycloaliphatische Alkylreste mit mindestens 8 bis 18 Kohlenstoffatomen handelt,
c) Vinylester von aliphatischen C₈- bis C₃₀-Carbonsäuren,
d) C₈- bis C₁₈-Alkyl-vinylether,
C) mehr als 0 bis höchstens 49 Gew.-% mindestens einem Comonomeren ausgewählt aus den Gruppen e) bis h) oder auch Mischungen verschiedenen Monomeren aus den Gruppen e) bis h)
e) C₁ bis C₄-Alkylester einer monoethylenisch ungesättigte Carbonsäure C,
f) N-C₁- bis C₄-Alkyl- oder N,N-C₁- bis C₄-Dialkyl-substituierte Carbonsäureamid einer monoethylenisch ungesättigte Carbonsäure C, wobei die Alkylreste des Dialkylamids gleich oder verschieden sein können,
g) Nitril einer monoethylenisch ungesättigte Carbonsäure C,
h) Acrylamidoglycolsäure, Vinylsulfonsäure, Allylsulfonsäure, Methallylsulfonsäure, Styrolsulfonsäure, Acrylsäure-(3-sulfo-propyl)ester, Methacrylsäure-(3-sulfopropyl)ester, Acryl-amidomethylpropansulfonsäure, Vinylphosphonsäure, Allylphosphonsäure, Acrylamidomethanpropanphosphonsäure, N-Vinylpyrrolidon, N-Vinylcaprolactam, N-Vinylimidazol, N-Vinyl-2-methylimidazol, N-Vinyl-4-methylimidazol, Diallylammoniumchlorid, Vinylacetat und Vinylpropionat,
wobei die Alkylgruppen mit Hydroxygruppen substituiert sein können und die monoethylenisch ungesättigte Carbonsäure C für die Gruppen e), f), g) unabhängig voneinander ausgewählt werden kann aus:
Acrylsäure, Methacrylsäure, Dimethacrylsäure, Ethacrylsäure, Maleinsäure, Citraconsäure, Methylenmalonsäure, Allylessigsäure, Vinylessigsäure, Crotonsäure, Fumarsäure, Mesaconsäure und Itaconsäure,
wobei sich die monoethylenisch ungesättigte Carbonsäure, die hydrophobe Komponente und die weiteren Monomeren zu 100% ergänzen,
zur Herstellung von Haarwaschmitteln

2. Verwendung von Copolymerisaten nach Anspruch 1, wobei die Komponente A eine C₃- bis C₈ monoethylenisch ungesättigte Carbonsäure ist und zu 65 bis 99 Gew.-% vorliegt.

3. Zusammensetzung, enthaltend:
a) wenigstens ein Alkyl- oder Alkenylpolyglykosid,
b) wenigstens ein Polymer nach einem der Ansprüche 1 oder 2,
c) gegebenenfalls wenigstens ein weiteres Tensid, und
d) gegebenenfalls wenigstens ein Neutralsalz.

4. Wäßrige Zusammensetzung nach Anspruch 3, enthaltend 0,2 bis 30 Gew.-% des Polymers b).

5. Zusammensetzung nach Anspruch 4, enthaltend:
a) 0,5 bis 60 Gew.-% eines Alkyl- oder Alkenylpolyglykosids,
b) 0,2 bis 30 Gew.-% eines Polymers nach einem der Ansprüchen 1 oder 2,
c) 1 bis 50 Gew.-% eines anionischen Tensides, und
d) 0,1 bis 5 Gew.-% eines Neutralsalzes,
bezogen auf das Gesamtgewicht der wäßrigen Formulierung.

6. Zusammensetzung nach einem der Ansprüche 3 bis 5, enthaltend:
a) 0,5 bis 50 Gew.-% eines Alkyl- oder Alkenylpolyglykosids,
b) 0,2 bis 20 Gew.-% eines Polymers nach einem der Ansprüche 1 oder 2,
bezogen auf das Gesamtgewicht der wäßrigen Formulierung.

7. Zusammensetzung nach Anspruch 6, enthaltend:
a) 0,5 bis 50 Gew.-% eines C₈-C₁₈-Alkyl- oder C₈-C₁₈-Alkenylpolyglykosids,
b) 0,5 bis 15 Gew.-% eines Polymers nach einem der Ansprüche 1 oder 2.

8. Zusammensetzung nach einem der Ansprüche 3 bis 7 in Form eines kosmetischen Mittels.

9. Verfahren zur Erhöhung der Viskosität von tensidhaltigen Zusammensetzungen, wobei man der Zusammensetzung eine verdickend wirkende Menge wenigstens eines Polymers nach einem der Ansprüchen 1 oder 2, zusetzt.

## Claims

1. The use of neutralized or partially neutralized copolymers obtainable from
A) from 50 to 99% by weight of monoethylenically unsaturated carboxylic acid and
B) from 1 to 50% by weight of at least one comonomer chosen from the groups a) to d) or also mixtures of different monomers from groups a) to d)
a) monoethylenically unsaturated carboxylic acid esters with a saturated C₈- to C₃₀-alcohol
b) N-alkyl- or N,N-dialkyl-substituted carboxamides, the alkyl radicals independently of one another being aliphatic or cycloaliphatic alkyl radicals having at least 8 to 18 carbon atoms,
c) vinyl esters of aliphatic C₈- to C₃₀-carboxylic acids,
d) C₈- to C₁₈-alkyl vinyl ethers,
C) more than 0 to at most 49% by weight of at least one comonomer chosen from groups e) to h) or else mixtures of different monomers from groups e) to h)
e) C₁- to C₄-alkyl ester of a monoethylenically unsaturated carboxylic acid C,
f) N-C₁- to C₄-alkyl- or N,N-C₁- to C₄-dialkyl-substituted carboxamide of a monoethylenically unsaturated carboxylic acid C, it being possible for the alkyl radicals of the dialkylamide to be identical or different,
g) nitrile of a monoethylenically unsaturated carboxylic acid C,
h) acrylamidoglycolic acid, vinylsulfonic acid, allylsulfonic acid, methallylsulfonic acid, styrenesulfonic acid, 3-sulfopropyl acrylate, 3-sulfopropyl methacrylate, acrylamidomethylpropanesulfonic acid, vinylphosphonic acid, allylphosphonic acid, acrylamidomethanepropanephosphonic acid, N-vinylpyrrolidone, N-vinylcaprolactam, N-vinylimidazole, N-vinyl-2-methylimidazole, N-vinyl-4-methylimidazole, diallylammonium chloride, vinyl acetate and vinyl propionate,
where the alkyl groups can be substituted with hydroxyl groups, and the monoethylenically unsaturated carboxylic acid C can be chosen independently of one another for groups e), f), g) from:
acrylic acid, methacrylic acid, dimethacrylic acid, ethacrylic acid, maleic acid, citraconic acid, methylenemalonic acid, allylacetic acid, vinylacetic acid, crotonic acid, fumaric acid, mesaconic acid and itaconic acid,
the monoethylenically unsaturated carboxylic acid, the hydrophobic component and the other monomers totaling 100%,
for the preparation of hair cleansers.

2. The use of copolymers according to claim 1, wherein component A is a C₃- to C₈-monoethylenically unsaturated carboxylic acid and is present in an amount of from 65 to 99% by weight.

3. A composition comprising:
a) at least one alkyl or alkenyl polyglycoside,
b) at least one polymer according to either of claims 1 and 2,
c) if appropriate at least one other surfactant, and
d) if appropraite at least one neutral salt.

4. The aqueous composition according to claim 3, comprising from 0.2 to 30% by weight of polymer b).

5. The composition according to claim 4, comprising:
a) from 0.5 to 60% by weight of an alkyl or alkenyl polyglycoside,
b) from 0.2 to 30% by weight of a polymer according to either of claims 1 and 2,
c) from 1 to 50% by weight of an anionic surfactant, and
d) from 0.1 to 5% by weight of a neutral salt,
based on the total weight of the aqueous formulation.

6. The composition according to one of claims 3 to 5, comprising:
a) from 0.5 to 50% by weight of an alkyl or alkenyl polyglycoside,
b) from 0.2 to 20% by weight of a polymer according to either of claims 1 and 2,
based on the total weight of the aqueous formulation.

7. The composition according to claim 6, comprising:
a) from 0.5 to 50% by weight of a C₈-C₁₈-alkyl or C₈-C₁₈-alkenylpolyglycoside,
b) from 0.5 to 15% by weight of a polymer according to either of claims 1 and 2.

8. The composition according to one of claims 3 to 7 in the form of a cosmetic product.

9. A process for increasing the viscosity of surfactant-containing compositions, where a thickening amount of at least one polymer according to either of claims 1 and 2 is added to the composition.

## Revendications

1. Utilisation de copolymères neutralisés ou partiellement neutralisés, que l'on peut obtenir à partir :
A) de 50 à 99 % en poids d'acide carboxylique à insaturation monoéthylénique, et
B) de 1 à 50 % en poids d'au moins un comonomère choisi parmi les groupements a) à d) suivants ou, également, de mélanges de différents monomères choisis parmi les groupements a) à d) :
a) des esters d'acide carboxylique à insaturation monoéthylénique avec un alcool saturé en C₈-C₃₀,
b) des amides d'acide carboxylique à substitution N-alkyle ou N,N-dialkyle, dans lesquels les radicaux alkyle représentent, indépendamment les uns des autres, des radicaux aliphatiques ou cyclo-aliphatiques ayant au moins 6 à 18 atomes de carbone,
c) des esters vinyliques d'acides carboxyliques aliphatiques en C₈-C₃₀,
d) des vinyléthers d'alkyle en C₈-C₁₈,
C) de plus de 0 % en poids jusqu'à une valeur maximale de 49 % en poids d'au moins un comonomère choisi parmi les groupements e) à h) suivants ou, également, de mélanges de différents monomères choisis parmi les groupements e) à h) :
e) des esters d'alkyle en C₁-C₄ d'un acide carboxylique à insaturation monoéthylénique C,
f) des amides d'acide carboxylique à substitution N-alkyle en C₁-C₄ ou N,N-dialkyle en C₁-C₄ d'un acide carboxylique à insaturation monoéthylénique C, les radicaux alkyle du dialkylamide pouvant être identiques ou différents,
g) un nitrile d'un acide carboxylique à insaturation monoéthylénique C,
h) l'acide acrylamidoglycolique, l'acide vinylsulfonique, l'acide allylsulfonique, l'acide méthallylsulfonique, l'acide styrènesulfonique, le (3-sulfopropyl)ester d'acide acrylique, le (3-sulfopropyl)ester d'acide méthacrylique, l'acide acrylamidométhylpropanesulfonique, l'acide vinylphosphonique, l'acide allylphosphonique, l'acide acrylamidométhanepropanephosphonique, la N-vinylpyrrolidone, le N-vinylcaprolactame, le N-vinylimidazole, le N-vinyl-2-méthylimidazole, le N-vinyl-4-méthylimidazole, le chlorure de diallylammonium, l'acétate de vinyle et le propionate de vinyle,
dans lesquels les groupements alkyle peuvent être substitués par des groupements hydroxyle, et l'acide carboxylique à insaturation monoéthylénique C, pour les groupements e), f) et g), peut être choisi, indépendamment les uns des autres, parmi les composés suivants :
l'acide acrylique, l'acide méthacrylique, l'acide diméthacrylique, l'acide éthacrylique, l'acide maléique, l'acide citraconique, l'acide méthylène-malonique, l'acide allylacétique, l'acide vinyl-acétique, l'acide crotonique, l'acide fumarique, l'acide mésaconique et l'acide itaconique,
l'acide carboxylique à insaturation monoéthylénique, le composant hydrophobe et les autres monomères se complétant jusqu'à 100 %,
en vue de la fabrication de produits de lavage pour les cheveux.

2. Utilisation de copolymères selon la revendication 1, dans laquelle le composant A est un acide carboxylique à insaturation monoéthylénique en C₃-C₈ et est présent en quantité de 65 à 99 % en poids.

3. Composition comprenant :
a) au moins un alkylpolyglycoside ou un alcénylpolyglycoside,
b) au moins un polymère selon l'une quelconque des revendications 1 ou 2,
c) éventuellement, au moins un autre tensioactif, et
d) éventuellement, au moins un sel neutre.

4. Composition aqueuse selon la revendication 3, contenant 0,2 à 30 % en poids du polymère b).

5. Composition selon la revendication 4, comprenant :
a) 0,5 à 60 % en poids d'un alkylpolyglycoside ou d'un alcénylpolyglycoside,
b) 0,2 à 30 % en poids d'un polymère selon l'une quelconque des revendications 1 ou 2,
c) 1 à 50 % en poids d'un tensioactif anionique, et
d) 0,1 à 5 % en poids d'un sel neutre,
par rapport au poids total de la formulation aqueuse.

6. Composition selon l'une quelconque des revendications 3 à 5, comprenant :
a) 0,5 à 50 % en poids d'un alkylpolyglycoside ou d'un alcénylpolyglycoside
b) 0,2 à 20 % en poids d'un polymère selon l'une quelconque des revendications 1 ou 2,
par rapport au poids total de la formulation aqueuse.

7. Composition selon la revendication 6, comprenant :
a) 0,5 à 50 % en poids d'un alkylpolyglycoside en C₈-C₁₈ ou d'un alcénylpolyglucoside en C₈-C₁₈,
b) 0,5 à 15 % en poids d'un polymère selon l'une quelconque des revendications 1 ou 2,

8. Composition selon l'une quelconque des revendications 3 à 7, se présentant sous la forme d'un agent cosmétique.

9. Procédé pour augmenter la viscosité de compositions contenant des tensioactifs, selon lequel on ajoute à la composition une quantité à action épaississante d'au moins un polymère selon l'une quelconque des revendications 1 ou 2.
